(19) Europäisches Patentamt
European Patent Office
Office européen des brevets

(11) Publication number : **0 490 598 A1**

# (12) EUROPEAN PATENT APPLICATION

(21) Application number : **91311400.5**

(22) Date of filing : **06.12.91**

(51) Int. Cl.[5] : **C07D 513/08,** A61K 31/425, // (C07D513/08, 277:00, 273:00)

(30) Priority : **07.12.90 GB 9026658**

(43) Date of publication of application : **17.06.92 Bulletin 92/25**

(84) Designated Contracting States : **AT BE CH DE DK ES FR GB GR IT LI LU NL SE**

(71) Applicant : **PHARMA MAR, S.A.**
**Calle de la Calera 3, Poligono Industrial de Tres Cantos**
**E-28760 Tres Cantos, Madrid (ES)**

(72) Inventor : **Northcote, Peter T**
**University of Canterbury, Dep. of Chemistery**
**Christchurch 1 (NZ)**
Inventor : **Munro, Murray H.G.**
**University of Canterbury, Dep. of Chemistry**
**Christchurch 1 (NZ)**
Inventor : **Blunt, John W.**
**University of Canterbury, Dep. of Chemistry**
**Christchurch 1 (NZ)**
Inventor : **Gravalos, Dolores G.**
**c/ Maldonado n 63**
**ES-28006 Madrid (ES)**

(74) Representative : **Lamb, John Baxter et al**
**MARKS & CLERK 57-60 Lincoln's Inn Fields**
**London WC2A 3LS (GB)**

(54) **Novel antitumoral and antiviral compound.**

(57) The novel compound pateamine of formula

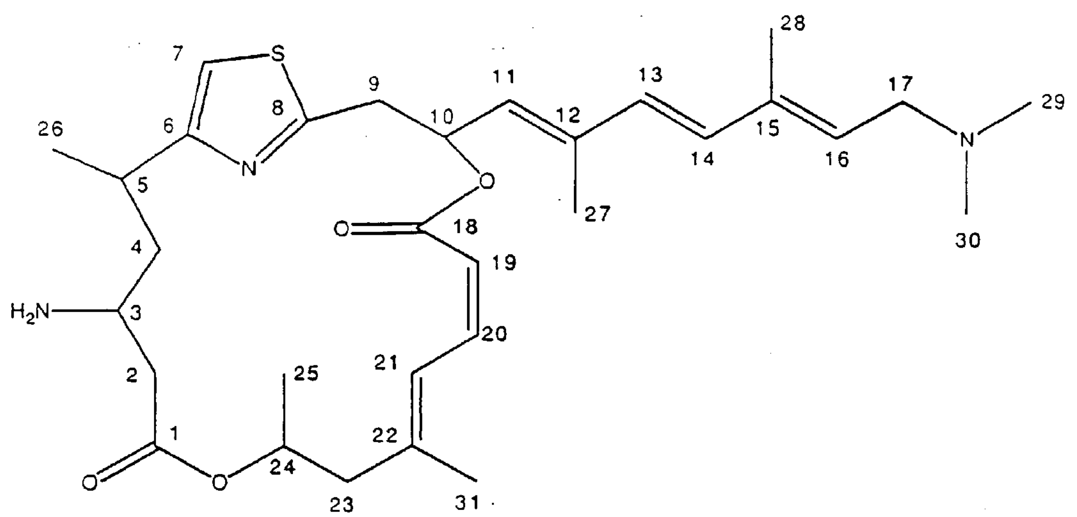

has antitumour and antiviral activity. Immunosuppressive activity is also present.

EP 0 490 598 A1

This invention relates to a novel antitumoral and antiviral compound which has been isolated from a sponge collected in Doubtful Sound, New Zealand.

The new compound, pateamine, has the structure and physical constants as below.

STRUCTURE

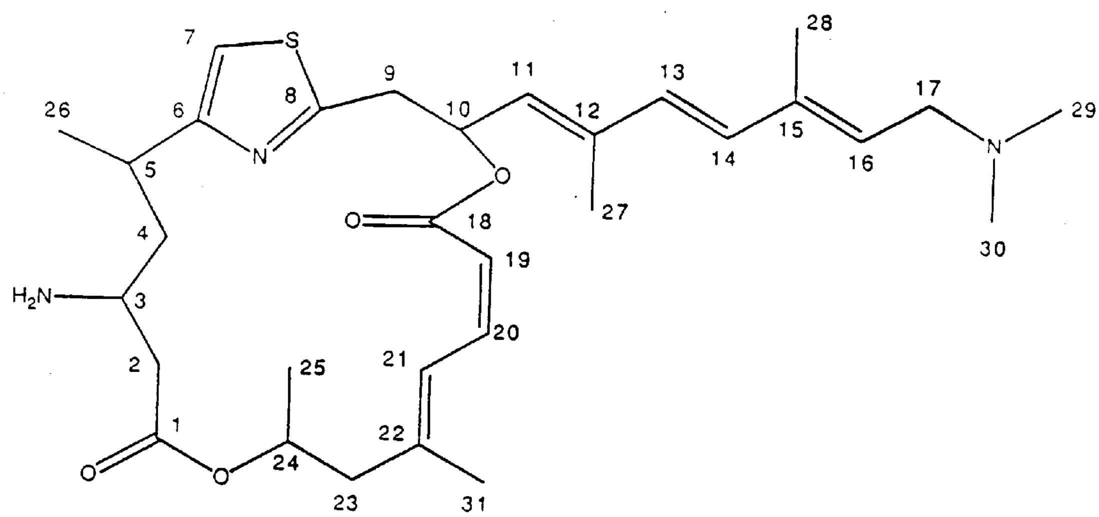

PHYSICAL CONSTANTS

Colourless Oil;
UV (MeOH) 206nm, 263nm (sh), 274nm, 285nm (sh);
IR (film) 2973, 2929, 2862, 2815, 2766, 1728, 1713, 1633, 1597, 1522, 1453, 1429, 1372, 1199, 1161, 1118, 1050, 1021, 979, 815 $cm^{-1}$;
$^1$H NMR (300MHz, $CDCl_3$) 7.00 (1H,d,11.7), 6.75 (1H, s), 6.68 (1H,t,11.6), 6.37 (1H,d,15.8), 6.25 (1H,dt,3.8, 8.7), 6.22 (1H,d,15.8), 5.75 (1H,t,7.2), 5.53 (1H,d,8.7), 5.41 (1H,d,11.6), 5.14 (1H,m), 3.2 (2H,m), 3.1 (2H,m), 3.05 (1H,m), 2.58 (1H,m), 2.44 (1H,m), 2.31 (1H,m), 2.25 (6H,s), 2.17 (1H,m), 2.11 (1H,m) 2.02 (3H,s), 1.95 (1H,m), 1.83 (3H,s), 1.81 (3H,s), 1.42 (1H,m), 1.27 (3H,d,7.2), 1.24 (3H,d,6.4);
$^{13}$C NMR (75 MHz,$CDCl_3$), 172.25 (C), 165.45 (C), 164.49 (C), 161.19 (C), 145.44 (C), 140.62 (CH), 138.11 (C), 135.92 (C), 134.01 (CH), 130.73 (CH), 130.25 (CH), 128.50 (CH), 123.84 (CH), 115.13 (CH), 112.41 (CH), 69.25 (CH), 67.26 (CH), 57.16 ($CH_2$), 48.19 ($Ch_2$), 45.48 (CH), 45.29 ($2XCH_3$), 45.12 ($CH_2$), 42.92 ($CH_2$), 38.86 ($CH_2$), 33.30 (CH), 22.79 ($CH_3$), 21.10 ($CH_3$), 16.80 ($CH_3$), 13.41 ($CH_3$), 12.68 ($CH_3$);
FABHRMS $M^+$+H 556.31523 ($C_{31}H_{46}0_4N_3S$ calc. 556.32087), $M^+$-$C_2H_6N$ 511.24245 ($C_{29}H_{39}0_4N_2S$ calc. 511.26303;
CILRMS ($NH_3$) 556(90), 512(10);
CILRMS ($ND_3$) 559(40);
DEILRMS 555(89), 512(37), 380(33), 176(100), 58(93).

ISOLATION

A sample of a Mycale sp of sponge (1Kg) collected in Doubtful Sound, New Zealand was extracted with methanol and a 3:1 mixture of methanol dichloromethane. An aqueous suspension of the entire 1 Kg extract of Mycale was extracted with three equivalent volumes of chloroform which was in turn back-extracted with distilled water. The organic extract (7.5g) was loaded onto a $C_{18}$ flash column (200g freshly prepared). Elution was started with 50% water/methanol, the methanol content was increased in steps to 100%, and then dichloromethane was added in gradual increments to 50%. Sixteen fractions were collected, weighed and assayed. Good total recovery of mass and activity was obtained with most of the activity confined to one fraction. The most active fraction (1.09g,$IC_{50}$2 ng/ml) was loaded onto a Diol flash column (50g). A gradient solvent sys-

tem was used ($CH_2Cl_2$,ETOAc, MeOH) and the fractions were monitored TLC. Much of the fatty acids and pigments were eluted with the mixtures of $CH_2Cl_2$/ETOAc, while a yellow charring ($H_2SO_4$ on silica) material came off in 5 - 20 % MeOH in ETOAc. The fractions containing the yellow char were combined to yield a mass of 240mg. This material was found to be active against the P388 cell line with an activity of 1.5 nanog/ml. This fraction was further purified using amino cartridges (500mg). The activity was eluted with MeOH to yield 45mg of material ($IC_{50}$0.5nanog/ml P388). Final purification was obtained on a LH20 column using the eluting solvent system 9:1 MeOH/$CH_2Cl_2$ with 2% conc. aqueous ammonia. This was selected to keep the active component in a free base form. Two of the six fractions containing the majority of the yellow charring compound were selected as the most pure on the basis of TLC and combined to yield a 17mg sample (PNI 62.1).

Physical properties, such as NMR spectra, were carried out on this, the free base form of pateamine ($IC_{50}$ 0.15nanog/ml P388). From the physical data the working structure has been elucidated.

NMR DATA

| POSITION | $^1H$ | | | | 13C |
|---|---|---|---|---|---|
| # | ppm | mult. | coupling | nOe to | |
| 1 | - | - | - | - | 172.25 |
| 2a | 2.44 | m | 2b,3 | - | 42.92 |
| 2b | 2.17 | m | 2a,3 | - | |
| 3 | 2.58 | m | 2a,2b,4a,4b | 4a | 45.48 |
| 4a | 1.95 | m | 3,4b,5 | 4b | 45.12 |
| 4b | 1.42 | m | 3,4a | 4a | |
| 5 | 3.05 | m | 4a,26 | 4b,7,26 | 33.30 |
| 6 | - | - | - | - | 161.19 |
| 7 | 6.75 | s | - | 5 | 112.41 |
| 8 | - | - | - | - | 165.45 |
| 9 | 3.2 | 2H,m | 10 | - | 38.86 |
| 10 | 6.25 | dt,3.8,8.7 | 9,11 | - | 69.25 |
| 11 | 5.53 | d,8.7 | 10.27 | 9,13 | 128.50 |
| 12 | - | - | - | - | 138.11 |
| 13 | 6.22 | d,15.8 | 14 | - | 130.73* |
| 14 | 6.37 | d,15.8 | 13 | 16.27 | 134.01 |
| 15 | - | - | - | - | 135.92 |
| 16 | 5.75 | t,7.2 | 17,28 | 14,17 | 130.25* |
| 17 | 3.1 | 2H,m | 16 | 28 | 57.16 |
| 18 | - | - | - | - | 164.49 |
| 19 | 5.41 | d,11.6 | 20 | 20 | 115.13 |
| 20 | 6.68 | t,11.6 | 19,21 | 19,31 | 140.62 |
| 21 | 7.00 | d,11.7 | 20,31 | - | 123.84 |
| 22 | - | - | - | - | 145.44 |
| 23a | 2.31 | m | 23b,24 | - | 48.19 |
| 23b | 2.11 | m | 23a | - | |
| 24 | 5.14 | m | 23a,25 | 23a,25 | 67.26 |
| 25 | 1.24 | 3H,d,6.4 | 24 | - | 21.10 |
| 26 | 1.27 | 3H,d,7.2 | 5 | - | 22.79 |
| 27 | 2.02 | 3H,s | 11 | 10.14 | 13.41 |
| 28 | 1.81 | 3H,s | 16 | - | 12.68 |
| 29 | 2.25 | 6H,s | - | 16,17 | 45.29 |
| 30 | " | " | - | " | " |
| 31 | 1.83 | 3H,s | 21 | - | 16.80 |

*these carbons can be exchanged

## Tests on pateamine

Pateamine was diluted in MeOH/$CH_2Cl_2$ 1:1 at 100 ng/ml and it has been tested at different concentrations. The solvent was allowed to evaporate before the cells were seeded.

Four general anitumoural assays, employing the current screening protocols have been carried out using the following cells:

P-388 (lymphoid neoplasm from DBA/2 mouse)

A-549 (human lung carcinoma)

HT-29 (human colon carcinoma)

KB (human oral epidermoid carcinoma)

It has been done two kinds of cytotoxicity assays, one of them employing the same screening protocols and another one with the MTT method. In both cases the cells were:

CV-1 (monkey kidney fribroblast)

BHK (hamster kidney fibroblast)

Two general antiviral assays, employing the current screening protocols have been carried out using the following virus:

HSV-1 (herpes simplex virus, type 1) in CV-1 cells

VSV (vesicular stomatitis virus) in BHK cells

The activity is measured by the following criteria:

The sign (-), indicates no inhibition of virus replication.

The sign (+), indicates that plaques are reduced by approx. 50-60%.

The sign (++), indicates that plaques are reduced by approx. 70-90%.

The sign (+++), indicates that plaques are reduced 100%.

(+) is the approximately $IC_{50}$

Numbers, indicate the cytotoxicity for the cells, which is determined by the inhibition zone on the cell sheet.

ANTITUMORAL ASSAYS

P-388 cells were seeded into 16 mm wells at 1 x $10^4$ per well in 1 mo aliquots of MEM 10FCS containing the indicated concentration or drug. All determinations were carried out in duplicate. After three days of incubation cells were counted and the $IC_{50}$ was determined.

DATA TABLE-I

% Inhibition P-388 cell growth

| Compound | 10ng | 5ng | 1ng | 0.5ng | 0.1ng | 0.5ng | $IC_{50}$ ng/ml approx. |
|---|---|---|---|---|---|---|---|
| PATEAMINE | 100 | 100 | 100 | 89 | 11 | 0 | 0.3 |

A-549 cells were seeded into 16 mm wells at 2 x $10^4$ per well. The day after the inoculum was replaced by 1 ml aliquots of MEM 10FCS containing the indicated concentration of drug. All determinations were carried out in duplicate. After three days of incubation cells were counted and the $IC_{50}$ was determined.

DATA TABLE-II

% Inhibition A-549 cell growth

| Compound | 10ng | 5ng | 1ng | 0.5ng | 0.1ng | 005ng | $IC_{50}$ ng/ml approx. |
|---|---|---|---|---|---|---|---|
| PATEAMINE | 100 | 100 | 71 | 32 | 0 | 0 | 0.7 |

HT-29 cells were seeded into 16 mm wells at 4 x $10^4$ per well. The day after the inoculum was replaced by 1 ml aliquots of MEM 10FCS containing the indicated concentration of drug. All determinations were carried out in duplicate. After four days of incubation cells were counted and the $IC_{50}$ was determined.

DATA TABLE-III

% Inhibition HT-29 cell growth

| Compound | 10ng | 5ng | 1ng | 0.5ng | 0.1ng | 0.05ng | $IC_{50}$ ng/ml approx. |
|---|---|---|---|---|---|---|---|
| PATEAMINE | 91 | 85 | 35 | 0 | 0 | 0 | 2 |

KB cells were seeded into 16 mm wells at 2 x $10^4$ cells per well. The day after the inoculum was replaced by 1 ml aliquots of MEM 10FCS containing the indicated concentration of drug. All determinations were carried out in duplicate. After four days of incubation cells were counted and the $IC_{50}$ was determined.

DATA TABLE-IV

% Inhibition KB cell growth

| Compound | 10ng | 5ng | 1ng | 0.5ng | 0.2ng | 0.05ng | $IC_{50}$ ng/ml approx. |
|---|---|---|---|---|---|---|---|
| PATEAMINE | 100 | 100 | 96 | 87 | 70 | 67 | <0.05 |

CYTOTOXICITY ASSAYS

CV-1 cells were seeded into 16 mm wells at 2 x $10^4$ per well. The day after the inoculum was replaced by 1 ml aliquots of MEM 10FCS containing the indicated concentration of drug. All determinations were carried out in duplicate. After four days of incubation cells were counted and the $IC_{50}$ was determined.

DATA TABLE-V

% Inhibition CV-1 cell growth

| Compound | 10ng | 5ng | 1ng | 0.5ng | 0.1ng | 0.05ng | $IC_{50}$ ng/ml approx. |
|---|---|---|---|---|---|---|---|
| PATEAMINE | 100 | 84 | 31 | 11 | 0 | 0 | 2.4 |

BHK cells were seeded into 16 mm wells at 2 x $10^4$ per well. The day after the inoculum was replaced by 1 ml aliquots of MEM 10FCS containing the indicated concentration of drug. All determinations were carried out in duplicate. After three days of incubation cells were counted and the $IC_{50}$ was determined.

DATA TABLE-VI

% Inhibition BHK cell growth

| Compound | 10ng | 5ng | 1ng | 0.5ng | 0.1ng | 0.05ng | $IC_{50}$ ng/ml approx. |
|---|---|---|---|---|---|---|---|
| PATEAMINE | 100 | 98 | 0 | 0 | 0 | 0 | 3 |

CV-1 and BHK cells were seeded into 9 mm wells at 1 x $10^4$ cells per well (logaritmic phase) and at 2 x $10^5$ cells per well (stationary phase). The day after the inoculum was replaced by 200 ul aliquots of MEM 10FCS containing different concentrations of drug. All determinations were carried out in duplicate. After three days of incubation, 50 ul of medium containing 100 ug of MTT were added. Plates were incubated for two hours, medium was removed and 100 ul of isopropanol were added. The absorbances were determined with a microtiter reader.

DATA TABLE-VII

| Celulas | $IC_{50}$ ng/ml log. phase | stat. phase |
|---|---|---|
| CV-1 | 2.4 | >100 |
| BHK | 3.7 | >100 |

ANTIVIRAL ASSAYS

DATA TABLE-VIII

% Inhibition CV-1/HSV-1

| Compound | 400ng | 200ng | 100ng | 40ng | 20ng | 10 ng | $IC_{50}$ ng/disk |
|---|---|---|---|---|---|---|---|
| PATEAMINE | 16+++ | 16+++ | 14+++ | 12++ | 10 + | 6 - | 20 |

DATA TABLE-IX

| | % Inhibition BHK/VSV | | | | | | |
|---|---|---|---|---|---|---|---|
| Compound | 400ng | 200ng | 100ng | 40ng | 20ng | 10ng | $IC_{50}$ ng/disk |
| PATEAMINE | 16 ? | 16 ? | 16 ? | 16 ? | 16 ? | 14 - | >10 |

Pateamine also has immunosuppresive activity, as shown by the following testing for immunomodulatory activity.

IMMUNOMODULATORY ACTIVITY

Establishing Primary Lymphocyte Cultures

Murine lymphocytes were derived by splenectomy, under sterile conditions, from Balb/c and C57B1/6 mice sacrificed by cervical dislocation. Spleens were homogenized in 15 mM RPMI medium 1040, 87%; fetal calf serum, 10%; L-glutamine (200 mM), 2% and supplemented with 1% antibiotic-antimycotic solution (GIBCO) and 25 µg/ml gentamycin. Red blood cells were lysed by brief exposure to sterile, distilled water and subsequently reconstituted to normal tonicity with PBS (10x). The final cell preparation was enumerated, and the viability evaluated, by means of a hemacytometer using the trypan blue exclusion method.

Procedure for a Mixed Lymphocyte Reaction Assay

General Description

The MLR is an in vitro test of cellular immunity in which murine lymphocytes from genetically dissimilar animals are cocultured to mutually induce cell proliferation (i.e. two-way MLR method). Mixed cell suspensions, together with the test compound, are incubated for 96 hours and subsequently pulsed for 15 hours with tritiated-thymidine (3H-Thy), a radioisotopically labelled nucleic acid. Tritiated-thymidine is incorporated into the DNA of proliferating cells. Data from the MLR is generated as dpm of radioactivity which is then expressed as a percentage of cellular activity, relative to control, using the following reduced formula:

$$\frac{\text{Experimental value} - (\text{Balb/c} - \text{C57B1/6} - \text{Media})}{\text{Positive Control} - (\text{Balb/c} - \text{C57B1/6} - \text{Media})} \times 100$$

In addition, a 50% inhibition concentration (IC.50) value was interpolated for each test compound.

Method

Reconstituted compound at each of several concentrations was added in duplicate (10µl) into wells of a 96-well microtiter plate and evaporated to dryness at room temperature. Mixed lymphocytes from Balb/c (H-2d) and C57B1/6 (H-26) mice were cocultured at 2.5 x 10E6 cells/ml in the presence of sample, alone or separately with an assay standard, cyclosporin A. All microtiter plates were incubated in a 5% $CO_2$ incubator at 37°C for 96 hours and pulsed for 15 hours with 1µCi3H thymidine (20 Ci/mmol) per well. The contents of each well are filtered through a glass fiber filter strip using an INOTECH cell harvestor. The radioactivity from DNA-bound 3H thymidine was counted using a dry INOTECH counter. Results were calculated as described above.

Procedure for Lymphocyte Viability Assay

General Description

The LCV is an in vitro cytotoxicity assay for murine lymphocytes designed by the investigator. The LCV is

similar to the MLR with the exception that only one cell population (Balb/c) is incubated with the test compound to assess cytotoxicty as distinct from the antiproliferative effects of a test compound. That is, unlike the unambiguous determination of immunostimulation from MLR data, a judgement of immunosuppression cannot be immediately interpreted. Cytotoxicity (e.g. due to direct cell lysis) from a compound can occur which would alter viability determinations inherent in the generation of MLR data. To ensure that cytotoxicity to the lymphocytes does not account for low data values, a parallel assay is performed. The percent viable cells is determined by the MTT-thiazolyl blue method after a similar incubation in the MLR of lymphocytes with the test sample. MTT is a substrate converted by mitchondrial enzymes in living cells to a purple, water-soluble, formazan crystal. If there is no significant reduction in cell viability, as determined by this detection method, then damaged cells are probably not present for low data values generated in the MLR. Colorimetric data from the LeV is calculated as a percentage of viability, relative to control, using the following formula:

$$\frac{\text{Experimental value} - (\text{Nonspecific Color})}{\text{Positive Control} - (\text{Media})} \times 100$$

In addition, a concentration representing a 50% viability (IC.50) value was interpolated for each test compound.

Method

Lymphocytes were prepared as described previously, with the exception that cells from only one murine strain were prepared (Balb/c). Balb/c lymphocytes (2.5 x 10E6 cells/ml) were added to one set of duplicates and to controls. Media alone was added to a set of duplicate samples to provide no specific, color conversion data. Plates were incubated in a 5% $CO_2$ incubator at 37°C for 96 hours and pulsed with MTT for another 15 hours. Insoluble crystals were dissolved in isopropyl alcohol and the plates were read using a BIOTEK plate reader. Results were calculated as described above.

The results of immunomodulating testing for pateamine show that pateamine is a potent immunosuppressive compound with an $IC_{50}$ for the MLR of <1.5 ng/ml and $IC_{50}$ for the LcV of 4.1 µg/ml.

## Claims

1. Pateamine, of formula:

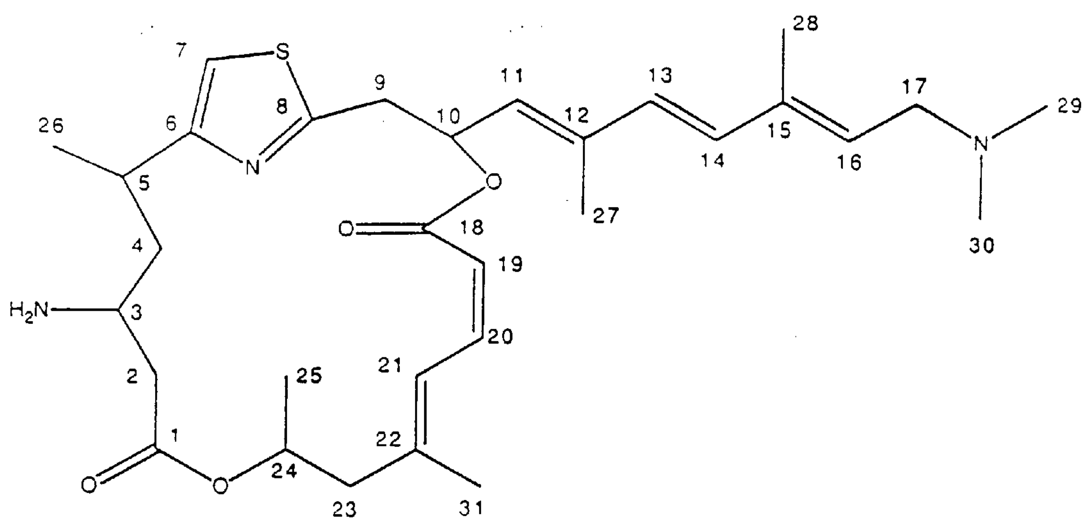

2. A compound conforming with the following spectral data:
UV (MeOH) 206nm, 263nm (sh), 274nm, 285nm (sh);
IR (film) 2973, 2929, 2862, 2815, 2766, 1728, 1713, 1633, 1597, 1522, 1453, 1429, 1372, 1199, 1161, 1118, 1050, 1021, 979, 815 $cm^{-1}$;

$^1H$ NMR (300MHz, $CDCl_3$) 7.00 (1H,d,11.7), 6.75 (1H, s), 6.68 (1H,t,11.6), 6.37 (1H,d,15.8), 6.25 (1H,dt,3.8, 8.7), 6.22 (1H,d,15.8), 5.75 (1H,t,7.2), 5.53 (1H,d,8.7), 5.41 (1H,d,11.6), 5.14 (1H,m), 3.2 (2H,m), 3.1 (2H,m), 3.05 (1H,m), 2.58 (1H,m), 2.44 (1H,m), 2.31 (1H,m), 2.25 (6H,s), 2.17 (1H,m), 2.11 (1H,m) 2.02 (3H,s), 1.95 (1H,m), 1.83 (3H,s), 1.81 (3H,s), 1.42 (1H,m), 1.27 (3H,d,7.2), 1.24 (3H,d,6.4);
$^{13}C$ NMR (75 MHz,$CDCl_3$), 172.25 (C), 165.45 (C), 164.49 (C), 161.19 (C), 145.44 (C), 140.62 (CH), 138.11 (C), 135.92 (C), 134.01 (CH), 130.73 (CH), 130.25 (CH), 128.50 (CH), 123.84 (CH), 115.13 (CH), 112.41 (CH), 69.25 (CH), 67.26 (CH), 57.16 ($CH_2$), 48.19 ($Ch_2$), 45.48 (CH), 45.29 ($2XCH_3$), 45.12 ($CH_2$), 42.92 ($CH_2$), 38.86 ($CH_2$), 33.30 (CH), 22.79 ($CH_3$), 21.10 ($CH_3$), 16.80 ($CH_3$), 13.41 ($CH_3$), 12.68 ($CH_3$);
FABHRMS $M^+$+H 556.31523 ($C_{31}H_{46}0_4N_3S$ calc. 556.32087), $M^+$-$C_2H_6N$ 511.24245 ($C_{29}H_{39}0_4N_2S$ calc. 511.26303;
CILRMS ($NH_3$) 556(90), 512(10);
CILRMS ($ND_3$) 559(40);
DEILRMS 555(89), 512(37), 380(33), 176(100), 58(93).

**3.** A pharmaceutical composition comprising the compound pateamine in combination with a pharmaceutically acceptable carrier or diluent.

**4.** A pharmaceutical composition comprising the compound of claim 2 in combination with a pharmaceutically acceptable carrier or diluent.

European Patent Office

# EUROPEAN SEARCH REPORT

Application Number

EP 91 31 1400

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | JOURNAL OF ORGANIC CHEMISTRY. vol. 53, no. 21, 14 October 1988, EASTON US pages 5014 - 5020; M. C. KERNAN ET AL.: 'Macrocyclic antifungal metabolites from the Spanish dancer nudibranch Hexabranchus sanguineus and sponges of the genus Halichondria' * Abstract; page 5017, compounds 6-11 * | 1,3 | C07D513/08 A61K31/425 //(C07D513/08, 277:00,273:00) |
| | ----- | | |
| | | | TECHNICAL FIELDS SEARCHED (Int. Cl.5 ) |
| | | | C07D A61K |

The present search report has been drawn up for all claims

| Place of search | Date of completion of the search | Examiner |
|---|---|---|
| THE HAGUE | 06 MARCH 1992 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)